Europäisches Patentamt

European Patent Office

Office européen des brevets

(11) Veröffentlichungsnummer: **0 134 536 B1**

## (12) EUROPÄISCHE PATENTSCHRIFT

(45) Veröffentlichungstag der Patentschrift: **25.03.92**

(21) Anmeldenummer: **84109138.2**

(22) Anmeldetag: **01.08.84**

(51) Int. Cl.⁵: **C12N 15/82**, C12N 5/04, A01H 1/02, A01H 4/00

(54) Verfahren zur Erhaltung und Vermehrung von defekten, nicht-infektiösen Virusgenomen.

(30) Priorität: **04.08.83 CH 4235/83**

(43) Veröffentlichungstag der Anmeldung:
**20.03.85 Patentblatt 85/12**

(45) Bekanntmachung des Hinweises auf die
Patenterteilung:
**25.03.92 Patentblatt 92/13**

(84) Benannte Vertragsstaaten:
**AT BE CH DE FR GB IT LI LU NL SE**

(56) Entgegenhaltungen:
**EP-A- 0 022 434**
**EP-A- 0 067 553**
**EP-A- 0 164 575**
**US-A- 4 407 956**

**Mol. Gen. Genet. Band 183, 1981, Seiten 209-213, Springer Verlag, DE, L. Otten et al. "Mendelian transmission of genes introduced into plants by the Ti plasmids of Agrobacterium tumefaciens"., Seite 184, rechte Spalte, erster Absatz**

(73) Patentinhaber: **CIBA-GEIGY AG**
**Klybeckstrasse 141**
**CH-4002 Basel(CH)**

(72) Erfinder: **Paszkowski, Jerzy, Dr.**
**Oberdorfstrasse 5**
**CH-4125 Riehen(CH)**
Erfinder: **Lazar, Gabor, Dr.**
**Genetikai Intezete TOB 521**
**H-6701 Szeged(HU)**
Erfinder: **Shinshi, Hideaki, Dr.**
**Holeeholzweg 61**
**CH-4102 Binningen(CH)**
Erfinder: **Rauseo, Isabelle, Dr.**
**Rue Franklin 69**
**F-68200 Mulhouse(FR)**
Erfinder: **Hohn, Thomas, Dr.**
**Talmattweg 11**
**CH-4103 Bottmingen(CH)**
Erfinder: **Potrykus, Ingo, Dr.**
**Im Stigler, 54**
**CH-4312 Magden(CH)**

(74) Vertreter: **Zumstein, Fritz, Dr. et al**
**Bräuhausstrasse 4**
**W-8000 München 2(DE)**

Basic life Sci., Band 26, 1983, Seiten 121-142. R.C. Gardner "Plant viral vectors : CaMV as an experimental tool", Seite 121, Absatz 2, Seite 122, Seite 131, letzter Absatz - Seite 132, Seite 138, Absatz 3.

Chemical abstracts, Band 89, 1978, Seite 286, Ref.Nr 56249t, Columbus, Ohio, US. S.H. Howell et al "Replication of cauliflower mosaic virus and transcription of its genome in trunip leaf protoplasts" & Virology, 1978, 86(2), 468-81

Nature, Band 294, Nr 5843, Dezember/Januar 1981/1982, Seiten 773-776, Chesham Bucks, GB. B. Gronenborn et al. "Prorogation of foreign DNA in plants using cauliflower mosaic virus as vector"

Nature, Band 293, Nr 5830, September 1981, Pages 265-270, Chesham, Bucks, GB. E.C. Cocking et al "Aspects of plant genetic manipulation", Seite 265, rechte Spalte, letzter Absatz, Seite 266, rechte Spalte, letzter Absatz, Seite 267, linke Spalte, Figur 1

Chemical abstracts, Band 98, 1983, Seite 132, Ref.Nr. 66277a, Columbus, Ohio, US. E. Balazs et al "Nucleotide sequence of DNA from an altered-virulence isolate D/H of the cauliflower mosaic virus" & Gene, 1982 19(3), 239-49

Chemical abstracts, Band 94. 1991, Seite 412, ref.Nr. 62040n, Columbus, Ohio, US. P. Brodelius et al "Entrapment of plant cells in different matrixes. A comparative study" & Febs Lett., 1980, 122(2), 312-16

Nature, Band 293, 8. Oktober 1981, Seiten 483-486, Chesham Bucks, GB. S.H. Howell et al "Rescue of in vitro generated mutants of clones cauliflower mosaic virus genome in infected plants"

Plant Molecular Biology, vol. 6, pages 303-312

Biological abstracts, Band 67, 1979, Ref. Nr. 56745, Philadelphia, US. T. Omura et al "Elimination of viruses from plant tissue cultures" & Ann. Phytopathol, Soc. Jpn, 44(3), 277-281, 1978

Biological abstracts, Band 77, Nr 8, 1984, Seite 6893, Ref.Nr. 62639, Philadelphia, US. H. Lorz et al "Improved protoplast culture and agarose media", & Plant cell tissue organ. Cult. 2(3), 217-226, 1983

Chemical abstracts, Band 100, 1984, Seite 264, Ref.Nr. 19990c, Columbus, Ohio, US. T.L. Adams et al "A new procedure for increasing efficiency of protoplast plating and clone selection" & Plant cell Rep., 1983, 2(4), 165-8

## Beschreibung

Die vorliegende Erfindung betrifft ein Verfahren zur Erhaltung und Vermehrung von defekten, nicht-infektiösen, Virusgenomen in proliferierendem, pflanzlichem Material.

Durch die Verwendung genetisch manipulierter Viren als Vektoren bei der Einführung von neuer genetischer Information in pflanzliches Erbgut kann pflanzliches Material mit neuen oder verbesserten Eigenschaften erzeugt werden.

Angesichts des raschen Anstiegs der Weltbevölkerung bildet die genetische Beeinflussung pflanzlichen Materials einen Schwerpunkt in der biologischen Forschung.

Auf der einen Seite steht die Suche nach alternativen, reproduzierbaren Nahrungsmittel-, Energie- und Rohstoffquellen wie z.B. neuen Pflanzen-, insbesondere Hybridsorten mit vorteilhaften Eigenschaften, beispielsweise einer erhöhten Resistenz gegenüber Krankheitserregern (wie phytopathogenen Insekten, Pilzen, Bakterien, Viren etc.), gegenüber Witterungs- oder Standorteinflüssen (wie Hitze, Kälte, Wind, Bodenbeschaffenheit, Feuchtigkeit, Trockenheit etc.) oder mit einer gesteigerten Reserve- und Vorratsstoffbildung in Blättern, Samen, Knollen, Wurzeln, Stengeln etc. Andererseits steigt neben der Nachfrage an wertvoller Biomasse auch der Bedarf an pharmazeutisch verwendbaren, pflanzlichen Wirksubstanzen und deren Abkömmlingen, wie z.B. Alkaloiden, Steroiden usw., die auf Grund ihrer geringen Ausbeuten aus natürlichen Vorkommen in zunehmendem Masse auf alternativem Weg, beispielsweise durch Extraktion aus genmanipulierten Pflanzensorten, zugänglich gemacht werden.

Es besteht daher ein steigendes Interesse an einer praktischen Möglichkeit der gezielten Manipulation von Pflanzen.

Ein Weg zu diesem Ziel ist die Uebertragung von Fremdgenen in isolierte Pflanzenzellen, die Replikation und Expression des genetischen Materials sowie seine Vermehrung und Erhaltung in allen sich durch Zellteilung aus der Ausgangszelle bildenden Tochterzellen. Solche Tochterzellen können in Form von Einzelzellen, Gewebekulturen oder ganzen Pflanzen vorliegen.

Im Stand der Technik sind bereits Verfahren beschrieben, die auf einen möglichen Einsatz von Viren als Vektoren für die Transformation pflanzlichen Materials abstellen. Howell SH und Hull R [Virology 86, 468-481 (1978)] beispielsweise beschreiben ein Kultivierungssystem für Protoplasten, das Studien über den Ablauf der Replikation bei Cauliflower Mosaik Viren [CaMV] erlaubt.

Gronenborn *et al* [Nature, 294, 773-776 (1981/82)] berichten über erste Transformationsversuche mit genetisch manipulierten CaMV Genomen, wobei die einzuschleusende Fremd-DNA gegen nich-essentielle, die Infektiösität der Viren nicht beeinflussende Abschnitte auf dem Virusgenom ausgetauscht wurde. Die manipulierten Viren behalten damit die für ihre Vermehrung als wesentlich angesehene volle Infektiösität. In die gleiche Richtung gehen auch diebei Cocking *et al* gemachten Vorschläge zum Einsatz von pflanzlichen DNA Viren als Transformationsvektoren.

Alle bisher beschriebenen oder vorgeschlagenen viralen Transformationssysteme haben aber gravierende Nachteile, die ihre allgemeine Anwendbarkeit in Frage stellen.

(a) So besteht beispielsweise bei sich teilenden pflanzlichen Zellen eine Tendenz, das Eindringen von Viren zu blockieren und die Lebens- und Vermehrungsfähigkeit von Viren zu hemmen oder vollständig zu unterbinden. Diese Tendenz kann fur bestimmte Viren durch den Einsatz geeigneter Kultivierungsbedingungen, die zur Ausbildung einer bestimmten Kallusform führen, bis zu einem gewissen Grad unterdrückt werden [Omura T und Wakimoto S, Ann. Phytopath. Soc. Japan 44,277-281(1978)].

Es ist aber beispielsweise bekannt, dass sich Cauliflower-Mosaic-Virus (CaMV) in den differenzierten Zellen einer Pflanze vermehren und alle weiteren differenzierten Zellen befallen kann, dass jedoch die Wachstumszentren oder Meristeme, d.h., die sich teilenden Zellen, nicht infiziert werden. So können von virusinfizierten Pflanzen über eine Meristemkultur virusfreie Pflanzen regeneriert werden.

(b) Genetisch manipulierte Viren, die Fremd-DNA im Austausch gegen nicht-essentielle, die Infektiosität nicht beeinflussende virale DNA Abschnitte enthalten, behalten ihre volle Pathogenität und führen daher zu den wohl bekannten Krankheitssymptomen bei den transformierten Pflanzen.

(c) Die Menge an Fremd-DNA, die in ein virales Genom im Austausch gegen nicht-essentielle, die Infektiosität nicht beeinflussende virale DNA Abschnitte eingebaut werden kann, ist starken Beschränkungen unterworfen aufgrund vorhandener Verpackungslimitierungen.

Es war daher zu erwarten, dass der Einsatz von Viren als Vektoren neuer genetischer Information nicht zu genetisch verändertem Pflanzenmaterial führen würde, da entweder die Viren nicht in proliferierendes, pflanzliches Material gelangen oder dort zugrunde gehen würden oder genetisches Material, welches über Cauliflower-Mosaic-Virus in proliferierende, pflanzliche Zellen eingebracht wird, nicht in das Erbgut der Pflanzenzellen aufgenommen oder nicht zur Replikation gelangen würde.

Man ging bisher davon aus, dass es für die Veränderung von pflanzlichem Erbgut unter Einsatz von Viren als Vektoren und zur Erzeugung genetisch identischer Nachkommenschaft des pflanzlichen Ausgangsmaterials erforderlich ist, Viren ohne Verlust ihrer Fähigkeit zur Selbstvermehrung und zum Eindringen in neue Wirtszellen zu kultivieren.

Es wurde nun gefunden, dass es überraschenderweise möglich ist, defekte, nicht infektiöse Virusgenome in sich teilendem, d.h. proliferierendem, pflanzlichem Material zu kultivieren und den Einbau von besagten defekten Virusgenomen in pflanzliche Genome zu erzielen.

Die vorliegende Erfindung betrifft daher ein Verfahren zur Erhaltung und Vermehrung von defekten, nicht-infektiösen Virusgenomen in proliferierendem, pflanzlichem Material, wobei dieses Verfahren darin besteht, dass man besagte defekte Virusgenome enthaltende, isolierte pflanzliche Protoplasten, Zellen oder Gewebe in einem geeigneten Nährmedium kultiviert. Hierbei ist auch die Kultivierung bis zur Regeneration vollständiger Pflanzen eingeschlossen. Die so erhaltenen Pflanzen erweisen sich in manchen Fällen als resistent gegen weitere Virusinfektionen (cross-resistance).

Im Rahmen des erfindungsgemässen Verfahrens lassen sich somit auch nicht-infektiöse, unter natürlichen Bedingungen nicht lebensfähige Viren (defekte Viren), welche im wesentlichen bezüglich der Grösse der eingebauten genetischen Information keine Begrenzung aufweisen, oder ihre Genome verwenden, wobei die Pflanzen, welche solche Viren oder ihre Genome enthalten, keine Krankheitssymptome zeigen. Daher lassen sich defekte Viren besonders vorteilhaft als Vektoren genetischer Informationen einsetzen. Ferner lassen sich aus Protoplasten, Zellen oder Geweben, welche defekte Viren oder deren Genome enthalten, vollständige Pflanzen regenerieren, welche gegen pathogene Viren resistent sind.

Die Packungsdichte der Virus-DNS in den Zellen infizierter Kalli ist sehr hoch und scheint weitgehend unbegrenzt zu sein. Die Virus-DNS liegt hier in freier, nicht von einer Proteinhülle umgebenen Form vor.

Im Rahmen der vorliegenden Erfindung gelten die folgenden Definitionen:

| | |
|---|---|
| Virusgenom: | Genetische Information eines Virus in Form ursprünglicher DNS ursprünglicher RNS eines Transcripts cDNS |
| Derivate eines Virusgenoms (defektes Virusgenom): | Deletionen Mutanten Neukombinationen Kombinationen mit anderem Genmaterial |
| Protoplast: | Isolierte, zellwandlose Pflanzenzelle mit der Potenz zur Regeneration in eine Zellkultur oder eine vollständige Pflanze |
| Zellkultur: | In undifferenziertem Zustand profilierende Masse von Zellen |

Als pflanzliches Ausgangsmaterial für das erfindungsgemässe Verfahren sind besonders isolierte Protoplasten, vor allem solche von Kulturpflanzen, geeignet.

Als Kulturpflanzen kommen hier vor allem solche der Familien Gramineae, Solanaceae, Compositae, Cruciferae und der Ordnung Leguminosae in Betracht.

Als Vertreter der vorgenannten Gruppe sind beispielsweise bei den Gramineen Weizen, Roggen, Gerste, Hafer, Reis, Mais, Hirse und Zuckerrohr, bei den Solanaceen Kartoffel, Tomate und Tabak, bei den Compositen Sonnenblume, Salat (Lactuca sativa), Endivie (Cichorium endivia) und Kamille (Matricaria), bei den Cruciferen verschiedene Kohl- und Rübenarten sowie Oel- und Gewürzpflanzen, und bei den Leguminosen Sojabohne, Lupine, Luzerne, Erbse, Bohne und Erdnuss zu nennen.

Unter den Cruciferen ist insbesondere die Gattung Brassica erwähnenswert, zu welcher beispielsweise Raps, Rübsen, Schwarzer und Weisser Senf sowie Kohl- und Rübenarten gehören, und hier ist besonders Brassica rapa, beispielsweise Brassica rapa cv Just Right, zu nennen.

Eine bevorzugte Ausführungsform des erfindungsgemässen Verfahrens besteht darin, dass man isolierte, pflanzliche Protoplasten, Zellen oder Gewebe kultiviert, welche Genome von defekten, nicht-infektiösen, Pflanzenviren enthalten.

Gemäss einer weiteren bevorzugten Ausführungsform des erfindungsgemässen Verfahrens enthalten die isolierten, pflanzlichen Protoplasten, Zellen oder Gewebe defekte, Virusgenome, welche genetisches Material enthalten, das in das Pflanzengenom der Protoplasten, Zellen oder Gewebe eingebaut wird.

Von den Viren sind insbesondere DNS-Viren, vor allem Caulimoviren, zu erwähnen und von diesen speziell Cauliflower-Mosaic-Virus (CaMV).

Als Virusgenome sind originäre Virus-DNS und DNS-Kopien von Virus-RNS hervorzuheben. Die defekten Virusgenome können durch genetische Manipulation entstehen, indern sie beispielsweise eingebautes, virusfremdes, genetisches Material enthalten.

Im allgemeinen ist es zweckmässig, isolierte, pflanzliche Protoplasten, Zellen und Gewebe zu kultivieren, welche defekte Virusgenome mit eingebautem, virusfremdem, genetischem Material pflanzlichen Ursprungs enthalten, und zwar vorzugsweise Material von einer Pflanze, deren Genom verschieden ist von

4

dem Genom derjenigen Protoplasten, Zellen oder Gewebe, welche die Virusgenome enthalten, d.h., dass als Empfänger und als Spender des genetischen Materials genetisch voneinander verschiedene Pflanzen fungieren.

Als Träger des eingebauten, virusfremden, genetischen Materials kommen vor allem DNS-Viren, vorzugsweise Caulimoviren, und von diesen insbesondere Cauliflower-Mosaic-Virus, in Betracht.

Eine besonders hervorzuhebende Ausführungsform des erfindungsgemässen Verfahrens besteht darin, dass man isolierte, pflanzliche Protoplasten, Zellen oder Gewebe von Brassica rapa, beispielsweise Brassica rapa cv Just Right, welche Genome von Cauli-flower Mosaic-Virus mit eingebautem, virusfremdem, genetischem Material von einer Pflanze enthalten, deren Genom von dem Genom der Protoplasten, Zellen oder Gewebe von Brassica rapa verschieden ist, kultiviert.

Die Gewinnung pflanzlicher, isolierter Protoplasten, Zellen oder Gewebe kann nach bekannten Methoden oder analog dazu erfolgen.

Isolierte pflanzliche Protoplasten, welche sich auch als Ausgangsmaterial für isolierte Zellen und Gewebe eignen, können von beliebigen Teilen der Pflanze gewonnen werden, wie beispielsweise von Blättern, Stengeln, Blüten, Wurzeln, Pollen oder Samen. Bevorzugt werden Blattprotoplasten verwendet. Die isolierten Protoplasten können auch aus Zellkulturen gewonnen werden. Methoden für die Isolierung von Protoplasten finden sich beispielsweise in Gamborg, O.L. und Wetter, L.R., Plant Tissue Culture Methods, 1975, 11-21.

Die Aufarbeitung und Kultivierung des pflanzlichen Materials erfolgt zweckmässigerweise gemäss den nachfolgenden Angaben, welche jedoch keine Beschränkung darstellen.

Die pflanzlichen Organe müssen - soweit es sich beim Ausgangsmaterial nicht bereits um sterile Zellkulturen handelt - sterilisiert werden, beispielsweise durch Behandlung mit Aethanol, $HgCl_2$, $Ca(OCl)_2$ oder kommerziellem Bleichungsmittel, und anschliessend in sterilem Wasser sorgfältig gewaschen werden. Alle folgenden Manipulationen müssen unter absolut sterilen Bedingungen vorgenommen werden, wobei Sterilität auch als Bedingung für eingesetzte Geräte und Lösungen gilt.

Der Abbau der Zellwände von Gewebeverbänden erfolgt enzymatisch, beispielsweise durch den Einsatz von Cellulasen, Hemicellulasen oder Pektinasen, welche kommerziell als mikrobielle Kulturfiltrate erhältlich sind und im Konzentrationsbereich von 0,001% bis 5% eingesetzt werden (soweit nicht anders angegeben, handelt es sich hier und im Folgenden um Gewichtsprozente).

Der Turgordruck der Pflanzenzellen muss während der Isolierung und der anschliessenden Kultur durch einen höheren osmotischen Druck des Isolierungsmilieus und des Kulturmediums ausgeglichen werden. Dies erfolgt durch den Einsatz osmotisch wirksamer Substanzen, wie beispielsweise Mannit, Sorbit, Saccharose, Glukose, $CaCl_2$ oder anderer Neutralsalzionen allein oder in Kombination untereinander und/oder mit einfachen Kulturmedien. Der osmotische Wert der Isolierungs- und Kulturmedien liegt zweckmässigerweise im Bereich von ca. 200 bis ca. 1000 mOs/kg $H_2O$ (Os = Molalität = Mol der osmotisch wirkenden Partikel/kg Lösungsmittel).

Die Enzymgemische werden im allgemeinen auf pH-Werte von 5,2 bis 8,0, insbesondere pH 5,4, eingestellt.

Die Inkubation der Gewebe, die zweckmässigerweise in feine Schnitte zerlegt werden (bei Blättern kann auch das Entfernen der Epidermis angewendet werden), erfolgt im allgemeinen bei Temperaturen im Bereich von 5 bis 40°C, zumeist bei 24°C. Die Inkubationsdauer richtet sich nach der Konzentration der Enzyme, der Inkubationstemperatur und dem Gewebetyp sowie dem Differenzierungszustand des Gewebes. Die Inkubationsdauer beträgt im allgemeinen zwischen 20 Minuten und mehreren Tagen.

Im Anschluss an die Freisetzung werden die Protoplasten über Siebe mit einer Maschenweite zwischen 25 und 280 $\mu$m von dem unverdauten Gewebe abgetrennt und durch Zentrifugieren konzentriert. Die Protoplasten sedimentieren oder flottieren in Abhängigkeit von ihrer eigenen Schwebedichte und der Dichte der Inkubations- bzw. Waschlösung. Die sedimentierenden oder flottierenden Protoplasten werden duch Resuspendieren und Zentrifugieren in osmotisch stabilisierten Waschmedien (beispielsweise Lösungen von Zuckeralkoholen, Zuckern, Neutralsalzen allein oder in Kombination untereinander und/oder mit Kulturmedien; pH im Bereich von 5,4 bis 6,5) wiederholt gewaschen und anschliessend in Kulturmedien aufgenommen. Ein wichtiger Faktor in der anschliessenden Kultur ist die Populationsdichte der Protoplasten, d.h. die Anzahl von Protoplasten pro Milliliter Kulturmedium. Die Populationsdichte liege zweckmässigerweise im Bereich von 1 bis 1 x $10^6$/ml, optimal ist jedoch ein Bereich von etwa 2 x $10^4$/ml bis 6 x $10^4$/ml.

Die Kulturtechniken variieren im allgemeinen von Kultur in flüssigem Kulturmedium als dünne Schicht in Petrischalen, über Tropfen oder hängende Tropfen mit Volumina im Bereich von etwa 1 $\mu$l bis 200 $\mu$l, bis zur Kultur in gelierten Kulturmedien (Agar, Agarose), oder in Kombinationen von gelierten mit flüssigen Kulturmedien. Die Kultivierung kann in Petrischalen, Multititerplatten, Dosen, Erlenmeyerkolben, Mikrokapillaren oder Gewebekulturflaschen, die aus beschichtetem oder unbeschichtetem Plastik oder aus Glas bestehen können, erfolgen.

Die Kultivierung lässt sich gewöhnlich in einem Temperaturbereich von 7° bis 42°C, vor allem aber im Bereich von 24 bis 27°C, durchführen. Die Temperatur kann entweder konstant gehalten werden oder sequenzweise variiert werden.

Die Lichtverhältnisse während der Kultivierung können zwischen Dauerdunkel und Dauerlicht im Bereich von 500 bis 5000 Lux und Licht-Dunkel-Folgen variieren. Vorzugsweise werden Licht-Dunkel-Folgen eingesetzt.

Es stehen bereits zahlreiche Kulturmedien zur Verfügung, die sich in einzelnen Medienkomponenten oder Gruppen solcher Komponenten unterscheiden. Alle Medien sind jedoch nach dem folgenden Prinzip aufgebaut: sie enthalten eine Gruppe anorganischer Ionen im Konzentrationsbereich von ca. 10 mg/l bis zu einigen Hundert mg/l (sogenannte Makroelemente wie beispielsweise Nitrat, Phosphat, Sulfat, Kalium, Magnesium, Eisen), eine weitere Gruppe anorganischer Ionen in Mengen von maximal einigen mg/l (die sogenannten Mikroelemente wie beispielsweise Kobalt, Zink, Kupfer, Mangan), ferner eine Reihe von Vitaminen (beispielsweise Inosit, Folsäure, Thiamin), eine Energie- und Kohlenstoffquelle wie beispielsweise Saccharose oder Glukose, sowie Wachstumsregulatoren in Form natürlicher oder synthetischer Phytohormone aus den Klassen der Auxine und Cytokinine im Konzentrationsbereich von 0,01 bis 10 mg/l. Die Kulturmedien sind zudem osmotisch stabilisiert durch Zusatz von Zuckeralkoholen (beispielsweise Mannit) oder Zucker (beispielsweise Glukose) oder Salzionen (beispielsweise $CaCl_2$) und sind auf einen pH-Wert von 5,6 bis 6,5 eingestellt.

Eine nähere Beschreibung gängiger Kulturmedien findet sich beispielsweise in Koblitz, H., Methodische Aspekte der Zell- und Gewebezüchtung bei Gramineen unter besonderer Berücksichtigung der Getreide, Kulturpflanze XXII, 1974, 93-157.

Die Kultivierungs- und Inkubationsbedingungen wie beispielsweise Temperatur des Nährmediums und der Umgebung, Luftfeuchtigkeit, Belichtungsintensität und -dauer, Ergänzung oder Erneuerung von Nährmedien oder Wechsel in ihrer Zusammensetzung, lassen sich den jeweiligen Gegebenheiten wie Art der Protoplasten, Art der Virusgenome oder ihrer Derivate, Wuchs- und Teilungsgeschwindigkeit des pflanzlichen Materials und/oder der Virusgenome, Menge an Ausscheidungsstoffen und anderen Faktoren anpassen.

Die Kultivierung der isolierten, pflanzlichen Protoplasten, Zellen oder Gewebe wird in einem geeigneten Nährmedium durchgeführt, im allgemeinen in einem gelierten Nährmedium.

Als Gelierungsmittel lässt sich Agarose mit besonders hervorragendem Erfolg einsetzen. Im allgemeinen beträgt der Gehalt an Agarose im Kulturmedium 0,4 bis 4%.

Agarose ist einer der Bestandteile des Agar. Käuflicher Agar besteht hauptsächlich aus einer Mischung von neutraler Agarose und ionischem Agaropectin mit einer Vielzahl gebundener Seitengruppen. Handelsübliche Agarose wird nach allgemein üblichen, kommerziellen Methoden aus Agar hergestellt. Im allgemeinen bleiben einige Seitengruppen erhalten und bestimmen im wesentllichen die physikochemischen Eigenschaften wie Gelbildung und Gelier- und Schmelztemperatur.

Bei niedrigen Temperaturen schmelzende und gelierende Agarose erzeugt man z.B. durch das nachträgliche Einführen von Hydroxyethylgruppen in die Agarosemoleküle. Diese derart modifizierte Agarose soll hier und im folgenden als LMT-Agarose (low melting agarose) bezeichnet werden.

Beispiele für geeignete Agarose-Präparationen sind die Typen Sea Plaque LMT, LMP, Typ VII, HGT, HGTP, LE Standard LMT oder Sea-Prep.

Die die defekten Virusgenome enthaltenden Protoplasten. Zellen oder Gewebe können auf verschiedene Weise in das Kulturmedium eingebracht werden. Man kann beispielsweise so vorgehen, dass man die die defekten Virusgenome enthaltenden Protoplasten, Zellen oder Gewebe in ein flüssiges, warmes Nährmedium einbringt, welches bereits das Gelierungsmittel enthält und welches beim Abkühlen erstarrt. Günstiger ist es jedoch, zu einer Suspension von den die defekten Virusgenome enthaltenden Protoplasten, Zellen oder Geweben in einem flüssigen Nährmedium ohne Gelierungsmittel eine weitere Portion flüssigen Nährmediums mit dem Gelierungsmittel zuzusetzen und nach gutem Durchmischen erstarren zu lassen.

Besonders gute Ergebnisse werden erzielt, wenn man das gelierte Nährmedium, in welchem sich die die defekten Virusgenome enthaltenden Protoplasten, Zellen oder Gewebe befinden, mit flüssigem Nährmedium umgibt. So kann beispielsweise das gelierte Nährmedium nach dem Ausbringen in Schalen und Platten, beispielsweise Petrischalen, wie sie auch zur Kultur von Bakterien oder Hefezellen verwendet werden, und nach dem Erstarren in Segmente zerlegt und diese in flüssiges Nährmedium, vorzugsweise in Nährlösung, eingebracht werden. Das Volumenverhältnis von Segmenten zum flüssigen Nährmedium bzw. der Nährlösung liegt zweckmässigerweise im Bereich von 1:20 bis 1:100. Es ist empfehlenswert, das flüssige Nährmedium mit den Segmenten in Bewegung zu halten, beispielsweise durch Schütteln auf einem Schüttelapparat. Die Segmentierung des gelierten Mediums erfolgt im allgemeinen unmittelbar nach dem Erstarren des Mediums oder innerhalb einer Frist von 4 Wochen, vorteilhafterweise 3 bis 8 Tage, nach dem Erstarren des Mediums.

Es werden Segmente hergestellt, welche vorzugsweise einheitlich in bezug auf Grösse und Form sind. Sie können eine unregelmässige oder bevorzugt regelmässige Ausdehnung aufweisen. Als Formen kommen beispielsweise Kegel, Kuben, Säulen, Prismen, Scheiben und Kugeln in Betracht. Die Segmente besitzen im allgemeinen einen mittleren Durchmesser oder Querschnitt von 2 bis 100 mm, insbesondere 2 bis 10 mm.

Im Rahmen des erfindungsgemässen Verfahrens können sowohl Protoplasten, Zellen und Gewebe eingesetzt werden, die bereits vor ihrer Isolierung mit Virus infiziert waren, wie auch solche, die bei ihrer Isolierung noch virusfrei waren und welche später artifiziell mit Virus infiziert werden.

Das erfindungsgemässe Verfahren betrifft somit in erster Linie, die Erhaltung und Vermehrung von Virusmutanten mit defekten Virusgenom, wobei die - die Virusgenome solcher Mutanten enthaltenden - isolierten, pflanzlichen Protoplasten, Zellen oder Gewebe in einem geeigneten Nährmedium kultiviert werden. Bei den Virus-Mutanten handelt es sich um solche, die unter natürlichen Bedingungen nicht lebensfähig sind, insbesondere um Mutanten von Pflanzenviren.

Die Protoplasten, Zellen oder Gewebe können beispielsweise originäre Virus-DNS oder DNS-Kopien der RNS von solchen Virus-Mutanten, die unter natürlichen Bedingungen nicht lebensfähig sind, oder entsprechende Mutanten solcher DNS-Viren enthalten, wobei als DNS-Viren insbesondere Caulimoviren, speziell Cauliflower-Mosaic-Virus, zu nennen sind.

Die vorliegende Erfindung erstreckt sich auch auf die Herstellung regenerierter, defekte Virusgenome enthaltender Pflanzenzellen, Pflanzengewebe oder ganzes Pflanzen, soweit sie aus isolierten, pflanzlichen, diese Virusgenome enthaltenden Protoplasten, insbesondere Protoplasten von Kulturpflanzen, erzeugt worden sind.

Als Kulturpflanzen kommen hier vor allem solche der Familien Gramineae, Solanaceae, Compositae, Cruciferae und der Ordnung Leguminosae in Betracht, wobei unter den Cruciferen insbesondere die Gattung Brassica zu erwähnen ist, und hier vor allem Brassica rapa, beispielsweise Brassica rapa cv Just Right.

Als Viren kommen insbesondere Pflanzenviren in Betracht.

Die regenerierten Pflanzenzellen, Pflanzengewebe oder ganze Pflanzen können beispielsweise originäre Virus-DNS, DNS-Kopien von Virus-RNS oder DNS-Viren enthalten, insbesondere Caulimoviren und speziell Cauliflower-Mosaic-Virus.

Besonders hervorzuheben sind regenerierte Pflanzenzellen oder -gewebe von Brassica rapa, welche defekte Cauliflower-Mosaic-Viren oder deren Genome enthalten.

Ferner können die regenierten Pflanzenzellen oder -gewebe solche defekten Virusgenome enthalten, die genetisches Material tragen, welches in das Genom der Pflanzenzellen oder -gewebe eingebaut wird, oder sie können genetisch manipulierte Virusgenome enthalten, beispielsweise solche, die eingebautes, virusfremdes, genetisches Material enthalten, das vorzugsweise von einer Pflanze stammt, deren Genom von demjenigen der Pflanzenzellen oder -gewebe, welche die defekten Virusgenome enthalten, verschieden ist. So können regenerierte Pflanzenzellen oder -gewebe beispielsweise Genome von Cauliflower-Mosaic-Virus mit eingebautem, virusfremdem, genetischem Material enthalten, insbesondere Material von einer Pflanze, deren Genom von demjenigen der Pflanzenzellen oder -gewebe, welche das defekte Cauliflower-Mosaic-Virus oder dessen Genom enthalten, verschieden ist.

Als Pflanze, welche das Cauliflower-Mosaic-Virus enthält, kommt hier insbesondere Brassica rapa, beispielsweise Brassica rapa cv Just Right, in Betracht.

Die vorliegende Erindung betrifft auch die Herstellung regenerierter Pflanzen, welche defekte Virusgenome enthalten und aus - diese Virusgenome enthaltenden - Protoplasten durch Kultivierung erzeugt worden sind, einschliesslich ihrer Nachkommenschaft, wobei die Nachkommenschaft sowohl durch sexuelle wie auch durch vegetative Vermehrung erzeugt worden sein kann.

Die vorliegende Erfindung schliesst ferner die Vermehrung von viralen in-vitro-Mutanten ein. Solche Mutanten werden vermehrt, indem man isolierte, pflanzliche Protoplasten mit genetisch manipulierten Viren oder Virusgenomen infiziert und in einem geeigneten Nährmedium, vorzugsweise einem durch Agarose gelierten Nährmedium, bis zur Ausdifferenzierung in eine Pflanze kultiviert.

Die vorliegende Erfindung umfasst weiterhin die Verwendung von viralen in-vitro-Mutanten, die wie vorstehend beschrieben unter Verwendung genetisch manipulierter Viren oder Virusgenome vermehrt worden sind, in Protoplasten-Transformationssystemen.

Die Virusvermehrung in aus Protoplasten entstandenen Zellkulturen lässt sich beispielsweise auf zwei verschiedenen Wegen nachweisen: a) durch Hybridisierung der Zellkultur-DNS mit radioaktiv markierter Virus-DNS oder b) durch Reinfektion gesunder Pflanzen mit Extrakten aus den Zellkulturen.

7

Methoden zur Isolierung von Protoplasten sowie zum Nachweis der Virusvermehrung sind bekannt. Es erweist sich jedoch als vorteilhaft, die Methoden den jeweiligen Gegebenheiten entsprechend zu variieren.

Nachfolgend wird die Gewinnung isolierter Protoplasten näher beschrieben, gefolgt von Beispielen für Protoplasten- und Zellvermehrung sowie für Nachweise der Virusvermehrung.

Pflanzen von Brassica rapa (Wasserrübe, Turnip) werden im Gewächshaus herangezogen und auf dem 5-Blattstadium mittels einer in der Virologie gängigen Methode (Einreiben von Virussuspensionen in Blattoberflächen durch mechanische Verletzung mit Carborundumpulver) mit Cauliflower-Mosaic-Virus infiziert. Systemisch infizierte Pflanzen werden in eine Pflanzenwuchskammer übertragen und dort unter folgenden Bedingungen weiterkultiviert: 12/12 Stunden Licht/Dunkel Rhythmus, 5000 lux Lichtintensität aus Leuchtstoffröhren SILVANIA daylight, 27°C Tages- und 20°C Nachttemperatur, 70% relative Luftfeuchtigkeit konstant, täglich zweimaliges Giessen der in Erde/Torf/Sandgemisch in Tontöpfen wachsenden Pflanzen mit 0,1% eines Pflanzendüngerkonzentrats, wie beispielsweise Greenzit®-Düngerlösung (CIBA-GEIGY AG, Basel). Ca. 12 cm lange Blätter der systemisch infizierten Pflanzen werden für die Protoplastenisolierung herangezogen: Die Blätter werden mit Leitungswasser gewaschen, für 30 Minuten in 0,5% Calciumhypochloridlösung sterilisiert und anschliessend sorgfältig unter sterilen Bedingungen in einer Sterilarbeitsbank fünfmal in sterilem entionisiertem Wasser gewaschen. Die Blattfläche wird in 2 cm breite Streifen zerlegt, welche gestapelt und mit absolut scharfen Rasierklingen in 1 mm breite Blattquerschnitte zerschnitten werden. Die Blattquerschnitte werden in eine osmotisch stabilisierte Enzymlösung folgender Zusammensetzung übertragen und diese Enzymlösung wird in das Gewebe vakuuminfiltriert: 1% Cellulase ONOZUKA R10$^R$ + 0,1% Pektinase MACEROZYME R10$^R$ (beides von Yakult Pharmaceutical Industry Co., Ltd. 8-21 Shingikan-cho, Nishinomiya, Japan) gelöst in einer Mischung von 0,45 molarem Mannit (3 Vol) und 0,17 molarem $CaCl_2$ (1 Vol), pH eingestellt auf 5,4 mit 0,1 molarer KOH. Der osmotische Wert der Enzymlösung beträgt ca. 510 mOs/kg $H_2O$. Während einer 16-stündigen Inkubation der Gewebeschnitte bei 12°C wird der überwiegende Anteil des Gewebes in isolierte Protoplasten zerlegt. Die Protoplastensuspension wird über ein 100 μm Stahlsieb von den unverdauten Gewebeteilen abgetrennt, in sterile Zentrifugenröhrchen übertragen und für 10 Minuten bei 750 x g sedimentiert. Die sedimentierten Protoplasten werden in sterilem Osmoticum (0,175 molares $CaCl_2$ + 0,5% MES*-Puffer, eingestellt auf pH 5,7, 510 mOs/kg $H_2O$) resuspendiert und durch Sedimentieren bei ca. 500 x g und Resuspendieren im Osmoticum dreimal gewaschen. Anschliessend werden die Protoplasten in dem nachfolgend unter Beispiel 1 angegebenen Kulturmedium resuspendiert und auf eine Populationsdichte von 8 x 10$^4$/ ml eingestellt.

Beispiel 1: Kultivierung von Protoplasten von Brassica rapa

\* MES:   2-(N-Morpholino)-äthansulfonsäure;          Sigma   No.   M-8250.

Kulturmedium:

modifiziert nach Kao, K.N. (1977), Molec.gen.Genet. 150, 225-230 und nach Koblitz K. and Koblitz, D. (1982), Plant Cell Reports 1, 147-150

| | | | | | |
|---|---|---|---|---|---|
| $NH_4NO_3$ | 600 | mg/l | Na-Pyruvat | 5 | mg/l |
| $KNO_3$ | 1900 | " | Citrat | 10 | " |
| $CaCl_2 \cdot 2H_2O$ | 600 | " | Malat | 10 | " |
| $MgSO_4 \cdot 7H_2O$ | 300 | " | Fumarat | 10 | " |
| $KH_2PO_4$ | 170 | " | Nicotinamid | 0,5 | " |
| KCl | 300 | " | Pyridoxin-HCl | 0,5 | " |
| $FeCl_3 \cdot 6H_2O$ | 27 | " | Thiamin-HCl | 5,0 | " |
| $Na_2EDTA$ | 74,6 | " | D-Calciumpantho-tenat | 0,5 | " |
| $MnSO_4 \cdot 1H_2O$ | 10 | " | Folat | 0,2 | " |
| $Na_2MoO_4 \cdot 2H_2O$ | 0,25 | " | p-Aminobenzoe-säure | 0,01 | " |
| $H_3BO_3$ | 3,0 | " | Biotin | 0,005 | " |
| $ZnSO_4 \cdot 7H_2O$ | 2,0 | " | Cholinchlorid | 0,5 | " |
| $CuSO_4 \cdot 5H_2O$ | 0,025 | " | Riboflavin | 0,1 | " |
| $CoCl_2 \cdot 6H_2O$ | 0,025 | " | Ascorbinsäure | 1,0 | " |
| KJ | 0,75 | " | Vitamin D3 | 0,005 | " |
| Fruktose | 125 | " | Vitamin B12 | 0,01 | " |
| Ribose | 125 | " | m-Inosit | 100 | |
| Xylose | 125 | " | Caseinhydro-lysat | 125 | " |
| Mannose | 125 | " | 2,4-Dichlorphen-oxyessigsäure | 0,25 | " |
| Rhamnose | 125 | " | α-Naphthylessig-säure | 0,5 | " |
| Cellobiose | 125 | " | 6-Benzylamino-purin | 0,1 | " |
| Sorbit | 125 | " | Saccharose | 20000 | " |
| Mannit | 125 | " | Glucose | 64500 | " |

pH (KOH) 5,8; 520 mOs/kg $H_2O$; alles gelöst und auf 1000 ml aufgefüllt mit quarzbidestilliertem Wasser.

Kulturmethode

Jeweils 2,5 ml der Protoplastensuspension in dem vorstehend angegebenen Kulturmedium werden in Plastikpetrischälchen von 6 cm Durchmesser pipettiert und dort mit dem gleichen Volumen des vorstehend angegebenen Kulturmediums, welches jedoch zusätzlich geschmolzene Agarose "Sea Plaque" (Marine Colloids) in einer Konzentration von 3% enthält, vermischt. Die Temperatur des Agarose enthaltenden Kulturmediums darf unmittelbar vor der Vermischung keinesfalls mehr als 40°C betragen. Nach dieser Behandlung sind die Protoplasten in einer Populationsdichte von $4 \times 10^4$/ml in dem gelierten Kulturmedium mit einem Agarosegehalt von 1,5% eingebettet. Im Anschluss an das Gelieren der Protoplastensuspension werden Sektoren aus dem Gel ausgeschnitten und in 30 ml des vorstehend angegebenen, flüssigen Kulturmediums in sterile Plastikdosen von 10 cm Durchmesser übertragen und auf einem Rotationsschüttler bei ca. 40 rpm in kontinuierlicher Dunkelheit und bei 24°C inkubiert. In Abständen von jeweils 3 bis 4 Tagen wird das flüssige Kulturmedium durch frisches Kulturmedium gleicher Zusammensetzung ersetzt.

Nach 14 tägiger Kultivierung wird der osmotische Wert des Kulturmediums durch schrittweises Vermindern des Osmoticums Glucose über einen Zeitraum von 3 Wochen auf 250 mOs/kg $H_2O$ herabgesetzt.

Bei dieser Behandlung entwickeln sich bis zu 40% der eingesetzten Protoplasten zu makroskopisch sichtbaren Zellkolonien.

Beispiel 2: Kultivierung von Zellkulturen von Brassica rapa

## Kulturmedium:

modifiziert nach Nitsch, J.P. and Nitsch, C. (1969), Science 163, 85-87

| | | | | | |
|---|---|---|---|---|---|
| $KNO_3$ | 950 | mg/l | m-Inosit | 100 | mg/l |
| $NH_4NO_3$ | 720 | " | Nicotinsäure | 5 | " |
| $MgSO_4 \cdot 7H_2O$ | 185 | " | Pyridoxin-HCl | 0,5 | " |
| $CaCl_2 \cdot 2H_2O$ | 220,5 | " | Thiamin-HCl | 0,5 | " |
| $KH_2PO_4$ | 68 | " | Folsäure | 0,5 | " |
| $FeCl_3 \cdot 6H_2O$ | 27 | " | Biotin | 0,05 | " |
| $Na_2EDTA$ | 74,6 | " | Glycin | 2 | |
| $MnSO_4 \cdot 1H_2O$ | 17,25 | " | Agar (Difco) | 9000 | " |
| $H_3BO_3$ | 10 | " | Saccharose | 20000 | " |
| $ZnSO_4 \cdot 7H_2O$ | 10 | " | Cocosmilch (Gibco) | 2,5 | " |
| $Na_2MoO_4 \cdot 2H_2O$ | 0,25 | " | 2,4-Dichlorphen-oxyessigsäure | 0,25 | " |
| $CuSO_4 \cdot 5H_2O$ | 0,025 | " | α-Naphthylessig-säure | 0,5 | " |
| | | | 6-Benzylaminopurin | 0,1 | " |

pH (KOH) 5,8; autoklaviert für 20 min bei 1 atü

Kulturmethode:

Zellkolonien aus Beispiel 1, die einen Durchmesser von 1 mm und mehr erreicht haben, werden auf die Oberfläche des vorstehend angegebenen, mit Agar gelierten Kulturmediums übertragen und weiter bei 24°C und Dauerdunkel inkubiert.

In der vorstehend beschriebenen Weise wurden in über 20 Versuchsserien Protoplasten aus nichtinfizierten und aus mit Cauliflower-Mosaic-Virus infizierten Brassica rapa-Pflanzen zu Zellkulturen regeneriert.

Beispiel 3: Nachweis der Virusvermehrung durch Hybridisierung der Zellkultur-DNS mit radioaktiv markierter Cauliflower-Mosaic-Virus-DNS

Wässrige Lösungen für die DNS-Hybridisierung:

| | |
|---|---|
| Proteinase K (Merck 24568): | 0,1 mg/ml Proteinase K 0,1% Natriumazid 0,1% Natriumdodecylsulfat (= SDS) |
| Alkali-Lösung: | 0,5 M NaOH 1,5 M NaCl |
| Neutralsalz-Lösung: | 3 M NaCl 0,5 M Tris(hydroxmethyl)-aminomethan-Puffer (= Tris-HCl-Puffer), pH 7,0 |

Methode

a) Einzelne Zellkolonien aus Beispiel 2 werden in je 1 ml 2-Methoxyäthanol 5 Stunden lang bei Raumtemperatur inkubiert;

b) das 2-Methoxyäthanol wird vorsichtig abgesaugt und die Zellkolonien in flüssigem Stickstoff gefroren;

c) die gefrorenen Zellkolonien werden in Eppendorfröhrchen zu Pulver homogenisiert;

d) das pulverisierte Material wird in je 200 µl $H_2O$ aufgeschwemmt und gut durchmischt;

e) anschliessend wird 2 Minuten scharf in der Eppendorf-Zentrifuge abzentrifugiert;

f) der klare Ueberstand wird für die DNS-Hybridisierung eingesetzt.

Hybridisierung:

g) je 50 µl des Ueberstands (s. Punkt f) werden in die Filterkammern des BRL-dot-hybridisations-Systems (Bethesda Research Laboratories BRL Nr. 1050 MM) übertragen, die Filter müssen vorher angefeuchtet werden und es ist ein mildes Vakuum (Wasserstrahlvakuum) erforderlich;

h) anschliessend werden je 200 µl Wasser hinzugegeben und durchfiltriert;

i) je 150 µl der vorstehend angegebenen Proteinase K-Lösung werden zu jeder Kammer gegeben, das System in Plastikfolie eingepackt, und über Nacht inkubiert;

j) die Proteinase-Lösung wird abgesaugt, durch 150 µl der vorstehend angegebenen Alkali-Lösung ersetzt und für 15 Minuten bei Raumtemperatur inkubiert;

k) die Alkali-Lösung wird abgesaugt und durch 150 µl der vorstehend angegebenen Neutralsalz-Lösung ersetzt und für weitere 15 Minuten inkubiert;

l) nach Absaugen der Neutralsalzlösung wird zweimal je 5 Minuten lang mit je 150 µl Natriumchlorid/Natriumcitrat-Puffer (= SSC: 17,5 g NaCl und 8,8 g Na-citrat/Liter $H_2O$; pH eingestellt mit NaOH auf 7) gewaschen;

m) nach dem Absaugen des SSC werden die Filter für 2 Stunden auf 80°C erhitzt; und

n) anschliessend mit radioaktiver CaMV-DNS hybridisiert;

o) radioaktiv-markierte Filter werden mit einem Film sichtbar gemacht;

p) Extrakte aus Zellkulturen, welche CaMV bzw. CaMV-DNS enthalten haben, hybridisieren auf dem Filter mit der radioaktiv markierten CaMV-DNS und geben damit eine Schwärzung des Films. Aus der Schwärzung oder Nichtschwärzung der Filter in den einzelnen Kammern des BRL-Hybrid-Dot-Systems nach photochemischer Behandlung lässt sich deshalb ablesen, welche der getesteten Zellkolonien CaMV bzw. CaMV-DNS enthalten hat.

Beispiel 4: Nachweis der Virusvermehrung durch Reinfektion gesunder Pflanzen mit Zellkultur-Extrakt

a) Einzelne Zellkolonien aus Beispiel 2 werden in je 1 ml 2-Methoxyäthanol 5 Stunden lang bei Raumtemperatur inkubiert.

b) Das 2-Methoxyäthanol wird vorsichtig abgesaugt und die Zellkolonien in flüssigem Stickstoff gefroren.

c) Die gefrorenen Zellkolonien werden in Eppendorfröhrchen zu Pulver homogenisiert.

d) Das pulverisierte Material wird in je 200µl $H_2O$ aufgeschwemmt und gut durchmischt.

e) Anschliessend wird 2 Minuten scharf in der Eppendorf-Zentrifuge abzentrifugiert.

f) Der klare Ueberstand wird für die Reinfektionsversuche eingesetzt.

Reinfektion:

Der Ueberstand (siehe Punkt f) wird unter strikten Sterilitätsbedingungen mit Carborundum in die Blattoberseite von gesunden Brassica rapa-Pflanzen eingerieben. Die Pflanzen werden für 3 bis 4 Wochen unter Ausschluss der Möglichkeit einer anderweitigen Virusinfektion gehalten und nach dieser Zeit auf das Auftreten von Virussymptomen hin untersucht (Mosaicsymptome, Transparentwerden der Blattadern). Mit Carborundum behandelte Kontrollpflanzen werden unter gleichen Bedingungen wie die inokulierten Pflanzen gehalten. Das Auftreten oder Ausbleiben von Virussymptomen nach dem Einreiben von gesunden Pflanzen mit dem Ueberstand erbringt den Nachweis der Gegenwart oder des Fehlens von funktionsfähigen CaMV-Partikeln in der Zellkultur, aus welcher der Ueberstand gewonnen wurde.

Die vorstehend beschriebenen Nachweise ergaben, dass in ca. 5% der Zellkulturen, die aus Protoplasten virusinfizierter Pflanzen regeneriert wurden, grosse Mengen an Virus gebildet worden sind.

Beispiel 5: Verwendung nicht-infektiöser Virusmutanten als Vektoren für Zellklone in Pflanzengeweben.

Saftproben von verschiedenen CaMV-DNS enthaltenden Kallus-Klonen von Brassica rapa werden jeweils in die Blattoberseite von gesunden Brassica rapa-Pflanzen eingerieben. Die Pflanzen werden für 3 bis 4 Wochen unter Ausschluss der Möglichkeit einer anderweitigen Virusinfektion gehalten und nach diesem Zeitraum auf das Vorliegen von Virussymptomen untersucht. Ausserdem wird Virus-DNS aus diesen Pflanzen isoliert.

50 % der Klone erweisen sich als infektiös und die Pflanzen, welche mit dem Saft dieser Klone behandelt worden sind, zeigen die üblichen Symptome einer CaMV-Infektion. Die Virus-DNS dieser Pflanzen weist ein Restriktionsmuster auf, welches identisch mit demjenigen der Virus-DNS der zur Behandlung verwendeten Saftprobe ist.

30 % der Klone erweisen sich als nicht-infektiös und eine DNS-Analyse zeigt, dass das Virusgenom Deletionen aufweist.

**Patentansprüche**

1. Verfahren zur Erhaltung und Vermehrung defekter, nicht-infektiöser Viren und/oder deren Genome in proliferierendem pflanzlichen Material, welches besagte defekten Viren oder viralen Genome enthält, die unter natürlichen Lebensbedingungen nicht länger lebensfähig sind, dadurch gekennzeichnet, dass man

(a) proliferierende, isolierte Protoplasten, Zellen oder Gewebe, welche defekte, nicht-infektiöse Viren oder deren Genome enthalten, in osmotisch stabilisierten Kulturmedien, die üblicherweise für die Kultivierung pflanzlicher Protoplasten, Zellen oder Gewebe verwendeten werden, kultiviert; und

(b) sofern gewünscht, besagte Protoplasten, Zellen oder Gewebe zu ganzen Pflanzen regeneriert.

2. Verfahren gemäss Anspruch 1, dadurch gekennzeichnet, dass die Dichte der proliferierenden, isolierten Protoplasten im Kulturmedium 1 bis $1 \times 10^6$/ml beträgt.

3. Verfahren gemäss Anspruch 1, dadurch gekennzeichnet, dass man die isolierten pflanzlichen Protoplasten, Zellen oder Gewebe, welche die defekten, nicht-infektiösen Viren oder deren Genome enthalten, bei einer Temperatur von 7°C bis 42°C kultiviert.

4. Verfahren gemäss Anspruch 1, dadurch gekennzeichnet, dass die Kultivierung der isolierten pflanzlichen Protoplasten, Zellen oder Gewebe, welche die defekten, nicht-infektiösen Viren oder deren Genome enthalten, in flüssigen Kulturmedien, über Tropfen oder hängenden Tropfen mit Volumina im Bereich von 1 μl lbis μl, in gelierten Kulturmedien oder in einer Kombination von gelierten und flüssigen Kulturmedien durchgeführt wird.

5. Verfahren gemäss Anspruch 4, dadurch gekennzeichnet, dass man die isolierten pflanzlichen Protoplasten, Zellen oder Gewebe, welche die defekten, nicht-infektiösen Viren oder deren Genome enthalten, in einem der üblicherweise für die Kultivierung pflanzlicher Protoplasten, Zellen oder Gewebe verwendeten Nährmedien einbringt, dieses Nährmedium mit Agarose geliert, das Nährmedium nach dem Erstarren segmentiert und die Segmentein eines der zuvor genannten Nährmedien einbringt.

6. Verfahren gemäss einem der Ansprüche 1 bis 5, dadurch gekennzeichnet, dass man pflanzliche Protoplasten, Zellen oder Gewebe verwendet, die bereits defekte, nicht-infektiöse Viren oder deren Genome enthalten.

**7.** Verfahren gemäss einem der Ansprüche 1 bis 5, dadurch gekennzeichnet, dass man pflanzliche Protoplasten, Zellen oder Gewebe, die bei ihrer Isolierung noch virusfrei sind, artifiziell mit defekten, nicht-infektiösen Viren oder deren Genomen inokuliert.

**8.** Verfahren gemäss einem der Ansprüche 1 bis 5, dadurch gekennzeichnet, dass der besagte Defekt auf eine Deletion, eine Mutation, eine Neukombination oder eine Kombination mit heterologem Genmaterial zurückgeht.

**9.** Verfahren gemäss einem der Ansprüche 1 bis 5, dadurch gekennzeichnet, dass es sich bei besagten defekten, nicht-infektösen Viren oder deren Gemomen um genetisch manipulierte Viren oder Virusgenome handelt.

**10.** Verfahren gemäss Anspruch 9, dadurch gekennzeichnet, dass das eingebaute, virus-fremde genetische Material heterologen Ursprungs ist im Verhältnis zum Genom des pflanzlichen Materials, das die defekten Viren oder deren Gemome enthält.

**11.** Verfahren gemäss einem der Ansprüche 1 bis 10, dadurch gekennzeichnet, dass besagte isolierte Protoplasten, Zellen oder Gewebe von Kulturpflanzen stammen.

**12.** Verfahren gemäss einem der Ansprüche 1 bis 10, dadurch gekennzeichnet, dass man besagte isolierte Protoplasten, Zellen oder Gewebe aus geeigneten Teilen der Pflanze ausgewählt aus der Gruppe bestehend aus Blättern, Stengeln, Blüten, Wurzeln, Pollen oder Samen isoliert.

**13.** Verfahren zur Herstellung von Pflanzen, die sich als resistent gegenüber weiteren Virusinfektionen erweisen, dadurch gekennzeichnet, dass man
(a) proliferierende, isolierte Protoplasten, Zellen oder Gewebe, welche defekte, nicht-infektiöse Viren oder deren Genome enthalten, in osmotisch stabilisierten Kulturmedien, die üblicherweise für die Kultivierung pflanzlicher Protoplasten, Zellen oder gewebe verwendeten werden, kultiviert; und
(b) besagte Protoplasten, Zellen oder Gewebe zu ganzen Pflanzen regeneriert.

## Claims

**1.** A process for the maintenance and proliferation of defective, non-infectious viruses and/or their genomes in proliferating plant material which contains said defective viruses or viral genomes which are no longer viable under natural conditions for life, which comprises
(a) culturing proliferating isolated protoplasts, cells or tissue, containing defective, non-infectious viruses or their genomes, in osmotically stabilised culture media which are usually used for culturing plant protoplasts, cells or tissue, and
(b) if desired, regenerating said protoplasts, cells or tissue to whole plants.

**2.** A process according to claim 1, wherein the density of the proliferating, isolating protoplasts in the culture medium is 1 to $1 \times 10^6$/ml.

**3.** A process according to claim 1, which comprises culturing the isolated plant protoplasts, cells or tissue containing the defective, non-infectious viruses or their genomes at a temperature from 7°C to 42°C.

**4.** A process according to claim 1, wherein the culturing of the isolated plant protoplasts, cells or tissue, containing the defective, non-infectious viruses or their genomes, is carried out in liquid culture media, via drops or suspended drops with a volume in the range from 1 $\mu$l to 200 $\mu$l, in gelated culture media or in a combination of gelated and liquid culture media.

**5.** A process according to claim 4, which comprises transferring the isolated plant protoplasts, cells or tissue, containing the defective, non-infectious viruses or their genomes, in one of the nutrient media which are usually used for culturing plant protoplasts, cells or tissue, gelating this nutrient medium with agarose, segmenting the nutrient medium after solidification and transferring the segments to one of the abovementioned nutrient media.

**6.** A process according to one of claims 1 to 5, which comprises using plant protoplasts, cells or tissue

EP 0 134 536 B1

already containing defective, non-infectious viruses or their genomes.

**7.** A process according to one of claims 1 to 5, which comprises inoculating plant protoplasts, cells or tissue, which upon isolation are still virus-free, artificially with defective, non-infectious viruses or their genomes.

**8.** A process according to one of claims 1 to 5, wherein the said defect originates from a deletion, a mutation, a recombination or a combination with heterologous genetic material.

**9.** A process according to one of claims 1 to 5, wherein said defective, non-infectious viruses or their genomes are genetically manipulated viruses or viral genomes.

**10.** A process according to claim 9, wherein the incorporated non-viral genetic material is of heterologous origin in relation to the genome of the plant material containing the defective viruses or their genomes.

**11.** A process according to one of claims 1 to 10, wherein said isolated protoplasts, cells or tissue are from cultivated plants.

**12.** A process according to one of claims 1 to 10, wherein said isolated protoplasts, cells or tissue are isolated from suitable parts of the plant, selected from the group comprising leaves, stems, flowers, roots, pollen or seeds.

**13.** A process for the production of plants which show resistance to further viral infections, which comprises
(a) culturing proliferating isolated protoplasts, cells or tissue, containing defective, non-infectious viruses or their genomes, in osmotically stabilised culture media which are usually used for culturing plant protoplasts, cells or tissue, and
(b) regenerating said protoplasts, cells or tissue to whole plants.

**Revendications**

**1.** Procédé d'obtention et de multiplication de virus déficients et non infectieux et/ou de leurs génomes dans du matériau végétal, proliférant, contenant lesdits virus déficients ou les génomes viraux qui, dans les conditions de vie naturelles, ne sont pas plus longtemps viables, caractérisé
(a) en ce que l'on utilise les protoplastes, cellules ou tissus isolés, proliférants, contenant des virus déficients et non infectieux ou leurs génomes, dans des milieux de culture osmotiquement stabilisés, lesdits milieux étant utilisés, de façon classique, pour cultiver des protoplastes, cellules ou tissus végétaux et,
(b) si désiré, en ce que l'on régénère lesdits protoplastes, cellules ou tissus en plantes entières.

**2.** Procédé selon la revendication 1, caractérisé en ce que la densité des protoplastes proliférants, isolés, dans le milieu de culture est comprise entre 1 et 1 x $10^6$/ml.

**3.** Procédé selon la revendication 1, caractérisé en ce que l'on cultive les protoplastes, cellules ou tissus végétaux, isolés, contenant les virus déficients et non infectieux ou leurs génomes, à une température allant de 7°C à 42°C.

**4.** Procédé selon la revendication 1, caractérisé en ce que la culture des protoplastes, cellules ou tissus végétaux, isolés, contenant les virus déficients et non infectieux, est effectuée dans des milieux de culture liquides, sur des gouttes ou gouttes pendantes ayant un volume compris entre 1 $\mu$l et 200 $\mu$l dans des milieux de culture gélifiés ou en combinant des milieux de culture gélifiés et liquides.

**5.** Procédé selon la revendication 4, caractérisé en ce que l'on introduit les protoplastes, cellules ou tissus végétaux, isolés, contenant les virus déficients et non infectieux ou leurs génomes, dans un des milieux nutritifs utilisés, de façon classique, pour la culture des protoplastes, cellules ou tissus végétaux, en ce que l'on gélifie ce milieu nutritif avec de l'agarose, en ce que l'on segmente le milieu nutritif après la solidification et en ce que l'on introduit les segments dans l'un des milieux nutritifs précédemment cités.

14

**6.** Procédé selon l'une des revendications 1 à 5, caractérisé en ce que l'on utilise des protoplastes, cellules ou tissus végétaux, contenant déjà des virus déficients et non infectieux ou leurs génomes.

**7.** Procédé selon l'une des revendications 1 à 5, caractérisé en ce que l'on inocule artificiellement avec des virus déficients et non infectieux ou avec leurs génomes, des protoplastes, cellules ou tissus végétaux, lesquels lors de leur isolement, ne contiennent pas encore de virus.

**8.** Procédé selon l'une des revendications 1 à 5, caractérisé en ce que ladite déficience remonte à une délétion, une mutation, une nouvelle combinaison ou une combinaison avec du matériau génétique hétérologue.

**9.** Procédé selon l'une des revendications 1 à 5, caractérisé en ce que lesdits virus déficients et non infectieux ou leurs génomes sont des virus ou des génomes viraux génétiquement manipulés.

**10.** Procédé selon la revendication 9, caractérisé en ce que le matériau génétique, étranger au virus et incorporé est d'origine hétérologue par rapport au génome du matériau végétal contenant les virus déficients ou leurs génomes.

**11.** Procédé selon l'une des revendications 1 à 10, caractérisé en ce que lesdits protoplastes, cellules ou tissus isolés proviennent de plantes de culture.

**12.** Procédé selon l'une des revendications 1 à 10, caractérisé en ce que l'on isole lesdits protoplastes, cellules ou tissus à partir de parties appropriées des plantes, choisies dans le groupe constitué par les feuilles, tiges, fleurs, racines, pollen ou semences.

**13.** Procédé pour la préparation de plantes se montrant résistantes vis-à-vis d'autres infections virales, caractérisé
(a) en ce que l'on cultive des protoplastes, cellules ou tissus proliférants, isolés, contenant des virus déficients et non infectieux ou leurs génomes, dans des milieux de culture osmotiquement stabilisés, lesdits milieux étant utilisés, de façon classique, pour la culture des protoplastes, cellules ou tissus végétaux; et
(b) en ce que l'on régénère lesdits protoplastes, cellules ou tissus en plantes entières.